# FASCICULE DE BREVET EUROPEEN

(11) **EP 1 433 460 B1**
(45) Date de publication et mention de la délivrance du brevet: **04.03.2009**
(21) Numéro de dépôt: 03293304.6
(22) Date de dépôt: 23.12.2003
(51) Int. Cl.: A61Q 1/02, A61K 8/04, A61K 8/19

(54) **Procédé de maquillage des peaux foncées**
Schminkvorgang für dunkle Haut
Make-up process for dark skin

(30) Priorité: 24.12.2002 FR 0216655
(43) Date de publication de la demande: 30.06.2004
(73) Titulaire: L'ORÉAL, 75008 Paris (FR)
(72) Inventeur: Lee, Hojung, 75005 Paris (FR); Soistier, Nicolas, 94270 Le Kremlin Bicetre (FR); Simon, Jean-Christophe, 162-0842 Tokyo (JP)
(74) Mandataire: Tanty, François

(56) Documents cités:
- EP-A1- 1 249 226
- EP-A1- 1 410 785
- EP-A2- 1 195 155
- EP-A2- 1 382 322
- EP-A2- 1 382 323
- WO-A1-02/56846
- DE-A1- 19 907 313
- FR-A1- 2 777 178
- US-A- 4 828 826
- US-A- 6 117 435
- US-A1- 2002 012 681

## Description

La présente invention concerne des procédés de maquillage comprenant l'application des compositions de type fond de teint destinées aux peaux foncées, y compris noires et métissées.

Au sens de la présente invention, une composition de type fond de teint désigne une composition de maquillage de la peau d'êtres humains. En l'occurrence, il peut s'agir d'un fond de teint à appliquer sur le visage ou le cou, d'un produit anti-cernes, d'un produit correcteur de teint, d'une crème teintée ou base de maquillage pour le visage ou d'une composition de maquillage du corps.

D'une manière générale, ce type de compositions contient, outre une phase grasse telle que cire ou huile, des charges minérales ou organiques et des agents de coloration.

Les agents de coloration sont utilisés à titre d'opacifiant et/ou de colorant et sont présents à une concentration suffisante pour procurer la couleur souhaitée.

En ce qui concerne les charges, elles sont classiquement utilisées pour modifier la rhéologie, ajuster la texture de la composition, en réduire la brillance et/ou à titre d'agent matifiant pour cacher les défauts cutanés de la surface à maquiller.

Les charges habituellement mises en oeuvre sont des particules de toutes formes, incolores ou blanches, minérales ou de synthèse et insolubles dans le milieu de la composition quelle que soit la température à laquelle la composition est fabriquée. Il s'agit généralement de silice, mica, talc, kaolin et plus particulièrement d'oxydes de titane. Ces charges sont classiquement utilisées à raison de 1 à 80 % et notamment de 1 à 50 % en poids, par rapport au poids total de la composition cosmétique.

WO 02/056846 décrit des compositions de type fond de teint comprenant des pigments à effet diffusant optique.

Toutefois, dans le cas des compositions de type fond de teint destinées aux peaux dites « foncées » et notamment de couleur noire, la présence de ces particules blanches peut être préjudiciable à l'effet esthétique recherché. En effet, ces particules blanches peuvent, dans certaines circonstances, liées notamment à leur proportion au sein de la composition et/ou à la couleur de la peau, générer un effet coloré grisâtre au niveau de la peau maquillée. Pour des raisons esthétiques évidentes, un tel effet est particulièrement indésirable.

La présente invention vise précisément à proposer des procédés de maquillage comprenant l'application des compositions de type fond de teint, notamment de maquillage de la peau, exemptes de cet effet secondaire et appropriées au maquillage des peaux foncées comme définies dans les revendications indépendantes et en particulier noires ou métissées.

En particulier, la présente invention vise à proposer des procédés de maquillage comprenant l'application des compositions de type fond de teint pour peaux foncées comme définies dans les revendications indépendantes qui avantageusement procure conjointement une uniformité de coloration entre les différentes zones du visage, notamment front, cernes, pommettes, et un effet éclaircissant quasi voire totalement exempt d'effet grisâtre. Il en ressort un effet de maquillage plus naturel du sujet maquillé.

La présente invention vise selon un de ses aspects, un procédé de maquillage d'une peau foncée comme définie dans la revendication 1, caractérisé en ce qu'il comprend l'application sur la peau d'au moins une composition de type fond de teint conforme à l'invention.

La présente invention concerne également, selon un autre de ses aspects, un procédé de maquillage des peaux foncées comme définies dans la revendication 23, comprenant l'application sur ladite peau d'au moins un produit de type fond de teint, comprenant au moins une première et une seconde compositions, chacune dans un contenant, la première composition comprenant, dans un premier milieu physiologiquement acceptable, au moins un agent de coloration conforme à l'invention et la seconde composition comprenant, dans un second milieu physiologiquement acceptable, au moins une nacre à titre de particules réfléchissantes.

En l'occurrence, le maquillage ainsi obtenu est un maquillage bicouche. L'ordre de superposition et/ou le mode de superposition des deux compositions, à savoir total ou partiel, peuvent par ailleurs être intéressants pour conférer des effets esthétiques supplémentaires.

Les inventeurs ont ainsi constaté qu'il était possible de mettre au point un maquillage résultant de l'application d'une composition cosmétique de type fond de teint dénuée d'effet coloré indésirable, en associant au moins un agent de coloration selon l'invention à des particules réfléchissantes de type de nacre, utilisées notamment en remplacement de charges conventionnelles, de manière partielle ou totale.

Par « peaux foncées », on désigne des peaux dont la clarté moyenne L* mesurée sur le front, les pommettes et le menton dans l'espace colorimétrique CIE 1976, est inférieure à 60 notamment à 55. La saturation C* peut être comprise par exemple entre 8 et 30, notamment entre 10 et 30, voire entre 12 et 28. Les valeurs d'angle de teinte h peuvent être comprises par exemple entre 38° environ et 77° environ, notamment entre 46° et 63°, par exemple entre 46° et 54°. Les valeurs de clarté L* peuvent être inférieures ou égales à 50, voire 45 ou 40 pour les peaux les plus sombres, tout en pouvant rester, pour la plupart des peaux, supérieures à 30. Des peaux foncées sont rencontrées par exemple parmi les populations africaine, afro-américaine, hispano-américaine, indienne et maghrébine.

D'une manière générale, les compositions de type fond de teint appliquées dans les procédés selon l'invention contiennent un agent de coloration ou un mélange d'agents de coloration en quantité suffisante pour leur conférer une teinte comprise dans la gamme de couleur s'étendant du beige rosé au brun orangé en passant notamment par le jaune orangé, un angle de teinte h variant de 40° à 70°, notamment de 50° à 70° et une saturation C* de 20 à 50. Ces paramètres sont caractérisés directement sur la composition selon le protocole présenté ci-après dans les exemples.

La composition de type fond de teint selon l'invention peut également être caractérisée par son spectre de réflectance. Ainsi, pour l'intervalle 600 à 680 nm, la réflectance varie de 10 à 45 %, et plus précisément de 12 à 40 %.

Par ailleurs, cette réflectance peut être inférieure à 20 % dans l'intervalle 450 nm à 500 nm.

L'ensemble de ces caractéristiques peut précisément être obtenu, dans le cadre de la présente invention, grâce à une combinaison d'agents de coloration spécifiques et de nacres à titre de particules réfléchissantes.

### Agents de coloration

Des agents de coloration convenant à l'invention produisent, seuls ou en mélange, une coloration jaune ou orangée. En d'autres termes, ils possèdent une réflectance significative dans l'intervalle 550 à 675 nm.

Le ou les agents de coloration peuvent être présents dans la composition de type fond de teint, notamment la composition de base ou de surface du produit de type fond de teint selon l'invention, à une teneur allant de 0,5 à 30 % en poids, notamment allant de 2 % à 20 % en poids et en particulier de 5 à 18 % en poids par rapport au poids total de la composition considérée.

Le ou les agents de coloration, peuvent être choisis parmi les pigments minéraux ou organiques, les polymères colorants, les colorants hydrosolubles ou liposolubles, les laques organiques, les poudres métalliques et leurs mélanges. Ils peuvent notamment être choisis parmi ceux cités dans le C.T.F.A Cosmetic Ingredient Handbook, 3ième Edition Cosmetic and fragrance Assn., Inc., Washington D.C. (1982).

A titre illustratif et non limitatif des agents de coloration minéraux, on peut plus particulièrement citer les oxydes métalliques jaunes, rouges, bruns comme par exemple les oxydes de fer.

Comme poudres métalliques, on peut citer la poudre de cuivre.

Conviennent notamment comme pigments organiques, les pigments FDC Yellow n° 5 (sel disodique de tartrazine).

A titre illustratif des laques organiques convenant à l'invention, on peut plus particulièrement citer les FDC Yellow n° 5 et n° 6 A1 Laque.

Les colorants hydrosolubles peuvent être choisis par exemple parmi le colorant brun identifié par l'appellation « caramel » selon le Color Index ; les colorants jaunes identifiés par les numéros de Color Index n° 10316, 13015, 18690, 18820, 18965, 19140, 45430, 47005, 75100 et celui dénommé Lactoflavine ; les colorants oranges identifiés par les numéros de Color Index n° 14270, 15510, 15980, 15985, 16230, 20170, 40215 ; les colorants rouges identifiés par les numéros de Color Index n° 14700, 14720, 14815, 15620, 16035, 16185, 16255, 16290, 17200, 18050, 18130, 18736, 24790, 27290, 45100, 45220, 45380, 45405, 45410, 45425, 45430, 75470 et leurs mélanges.

Les colorants liposolubles peuvent être choisis par exemple parmi le colorant brun identifié par le numéro Color Index n° 12010 ; les colorants jaunes identifiés respectivement par les numéros de Color Index n° 12700, 21230, 47000, 75125, 75135 ; les colorants oranges identifiés par les numéros de Color Index n° 11920, 40800, 40820, 40825, 40850, 45396, 75120, 75130 et la capasanthine et le colorant rouge identifié par le n° 12150 et leurs mélanges.

Le polymère colorant est un polymère comportant au moins un groupement colorant organique. Le groupement colorant peut être greffé, notamment par liaison covalente, sur la chaîne du polymère. Le polymère colorant contient en général moins de 10 % en poids, par rapport au poids total du polymère, de matière colorante.

Ce polymère colorant peut être de toute nature chimique, notamment polyester, polyamide, polyuréthanne, polyacrylique, poly(méth)acrylique, polycarbonate, d'origine naturelle comme les polymères cellulosiques ou de chitosane, ou un de leurs mélanges, et en particulier un polyester ou polyuréthanne.

En particulier, le polymère colorant peut être un copolymère à base d'au moins deux monomères distincts dont l'un au moins est un monomère colorant organique.

De tels polymères colorants sont notamment décrits dans les brevets ou demandes de brevet US 5 032 670, US 4 999 418, US 5 106 942, US 5 030 708, US 5 102 980, US 5 043 376, US 5 104 913, US 5 281 659, US 5 194 463, US 4 804 719, WO 92/07913 et EP-A-747036.

A titre illustratif de monomères pour polymères colorant connus, on peut notamment citer les anthraquinones, les méthines, bis-méthines, les aza-méthines, les arylidènes, les 3H-dibenzo[7,i-j] isoquinolines, les acides 2,5-diarylaminotéréphtaliques et leurs esters, les phtaloylphénothiazines, les phtaloylphénoxazines, les phtaloylacridone, les anthrapyrimidines, les anthrapyrazoles, les phtalocyanines, les quinophtalones, les indophénols, les perinones, les nitroarylamines, le benzodifurane, les 2 H-1-benzopyran-2-one, les quinophtalones, les perylènes, les quinacridones, les triphénodioxazines, les fluoridines, les 4-amino-1,8-naphtalimides, les thioxanthrones, les benzanthrones, les indanthrones, les indigo, thioindigo, xanthène, acridine, azine et oxazine.

L'homme de l'art est bien entendu à même, de par ses connaissances générales, de procéder aux choix des monomères pour ajuster l'effet de couleur recherché selon l'invention.

Le ou les agents de coloration et notamment les pigments mis en oeuvre dans le cadre de la présente invention, peuvent être utilisés soit sous leur forme brute ou sous une forme prétraitée, notamment en leur surface. Ce traitement a généralement pour objectif d'augmenter la stabilité de la couleur et faciliter leur incorporation dans les formulations cosmétiques. En particulier, des agents de coloration, traités en vu de les rendre hydrophobes seront plus facilement dispersibles dans une phase huileuse.

A titre représentatif de ces traitements de surface, on peut notamment citer celui consistant à traiter le pigment avec un agent hydrophobe et oléofuge de type dérivé phosphate perfluoroalkyle comme décrit dans le brevet EP 1 086 683.

De même, il peut être utile de traiter les agents de coloration et notamment les pigments avec un matériau qui les rend compatibles avec les phases huileuses et notamment siliconées utilisées dans les formulations cosmétiques. Des pigments de ce type sont notamment décrits dans le brevet US 5 143 722.

Selon un mode de réalisation particulier, les agents de coloration utilisés selon l'invention sont de couleur jaune, orange, brune ou rouge.

A titre illustratif des agents de coloration convenant plus particulièrement à l'invention, on peut notamment citer les oxydes de fer brun et de fer jaune, enrobés de phosphate de perfluoroalkyle et l'oxyde de titane traité alumine, enrobé de phosphate de perfluoroalkyle, ou l'oxyde de titane enrobé de phosphate de perfluoroalkyle, comme en particulier les pâtes pigmentaires commercialisées, sous les dénominations commerciales YELLOW IRON OXYDE COVAFLUOR, PF5 YELLOW 601 (jaune) et PF5 RED R516L (rouge), PF5 BLACK BL100 par la société DAITO, sous les dénominations commerciales FA50DRF, FA50DYF, FA65DF et FA65DBF par la société KOBO, le bleu d'outremer enrobé de phosphate de perfluoroalkyle sous la dénomination commerciale PF5 ULTRAMARINE n° 801 par la société DAITO, les sels disodiques de Tartrazine et les laques d'aluminium du rouge Allura sur alumine commercialisées par la société NOVEON sous les dénominations FDC YELLOW n° 6, A1 Laque et FDC YELLOW N° 5 A1 Laque et leurs mélanges.

### Particules réfléchissantes

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente. Cette réflexion peut, le cas échéant, posséder une intensité suffisante pour créer à la surface de la composition de type fond de teint selon l'invention, lorsque cette dernière est appliquée sur le support à maquiller, des points de surbrillance visibles à l'oeil nu, c'est-à-dire des points plus lumineux qui contrastent avec leur environnement en semblant briller.

Les particules réfléchissantes sont également sélectionnées de manière à ne pas altérer significativement l'effet de coloration généré par les agents de coloration qui leur sont associés et plus particulièrement de manière à optimiser cet effet en terme de rendu de couleur. Elles peuvent plus particulièrement posséder une couleur ou un reflet jaune, rose, rouge, bronze, orangé, brun, or et/ou cuivré.

Les particules réfléchissantes peuvent être présentes dans la composition selon l'invention et la composition de base ou de surface du produit selon l'invention, à une teneur allant de 0,5 % à 60 % par rapport au poids total de la composition, notamment de 1 % à 30 % en poids, en particulier de 2 % à 20 % en poids, voire de 3 % à 10 % en poids.

Ces particules peuvent présenter des formes variées. Ces particules peuvent être notamment en forme de plaquettes ou globulaires, en particulier sphériques.

Les particules réfléchissantes quelle que soit leur forme, peuvent présenter une structure multicouche ou non et, dans le cas d'une structure multicouche, par exemple au moins une couche d'épaisseur uniforme, notamment d'un matériau réfléchissant.

Lorsque les particules réfléchissantes ne présentent pas de structure multicouche, elles peuvent être composées par exemple d'oxydes métalliques, par exemple des oxydes de titane ou de fer obtenus par synthèse.

Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant notamment d'au moins un métal ou composé métallique. Le substrat peut être monomatière, multimatériau, organique et/ou inorganique.

Plus particulièrement, il peut être choisi par les verres, les céramiques, le graphite, les oxydes métalliques, les alumines, les silices, les silicates, notamment les aluminosilicates et les borosilicates, le mica synthétique et leurs mélanges, cette liste n'étant pas limitative.

Le matériau réfléchissant peut comporter une couche de métal ou d'un composé métallique.

Des particules de verre recouvertes d'une couche métallique sont décrites notamment dans les documents JP-A-09188830, JP-A-10158450, JP-A-10158541, JP-A-07258460 et JP-A-05017710.

Toujours à titre d'exemple de particules réfléchissantes comportant un substrat minéral enrobé d'une couche de métal, on peut citer également les particules comportant un substrat de borosilicate enrobé d'argent, encore appelées « nacres blanches ».

Des particules à substrat de verre revêtu d'argent, en forme de plaquettes, sont vendues sous la dénomination MICROGLASS METASHINE REFSX 2025 PS par la société TOYAL. Des particules à substrat de verre revêtu d'alliage nickel/chrome/molybdène sont vendues sous la dénomination CRYSTAL STAR GF 550, GF 2525 par cette même société.

Les particules réfléchissantes quelle que soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.

A titre d'exemple de telles particules, on peut citer par exemple les particules comportant un substrat de mica synthétique revêtu de dioxyde de titane, ou les particules de verre enrobé soit d'oxyde de fer brun, d'oxyde de titane, d'oxyde d'étain ou d'un de leurs mélanges comme celles vendues sous la marque REFLECKS^{®} par la société ENGELHARD.

Les particules réfléchissantes peuvent être ou non goniochromatiques et/ou interférentielles ou non. Elles comprennent au sens de l'invention, les nacres et les agents de coloration goniochromatiques.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica); les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

On peut également envisager d'utiliser à titre de particules réfléchissantes, un agent de coloration goniochromatique sous réserve que cet agent réponde à l'exigence d'effet de teinte requise selon l'invention et ne perturbe pas, par ailleurs, la perception visuelle de la composition en terme d'effet couleur. Cet agent de coloration goniochromatique peut être notamment choisi parmi les structures multicouche interférentielles.

### Charges

Comme précisé précédemment, la présence de particules réfléchissantes en proportion suffisante pour assurer une fonction de charge permet de réduire significativement la quantité voire d'éviter la présence de charge(s) conventionnelle(s) de type particules blanches.

En l'occurrence, les compositions de type fond de teint appliquées conformément à l'invention peuvent contenir moins de 5 % voire 3 % en poids en particules blanches et notamment d'oxyde de titane, voire peuvent être exemptes en oxyde de titane.

Bien entendu, il demeure toutefois possible d'associer aux particules réfléchissantes selon l'invention une ou plusieurs autres charges conventionnelles sous réserve que celles-ci soient utilisées en une quantité telle qu'elles ne viennent pas affecter l'effet esthétique recherché par les composition de type fond de teint revendiquées c'est-à-dire ne confèrent pas outre mesure un aspect grisâtre à la peau maquillée lorsque celle-ci est revêtue d'une composition conforme à l'invention. L'homme de l'art est à même, de par ses connaissances, de procéder à cet ajustement.

Ces charges peuvent être minérales ou organiques de toute forme, plaquettaires, sphériques ou oblongues, quelque soit la forme cristallographique (par exemple feuillet, cubique, hexagonale, orthorhombique, etc). On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning) ou de polyméthacrylate de méthyle (Covabead de Wackherr), les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium et leurs mélanges.

Il peut être avantageux de privilégier le choix de charges complémentaires, transparentes, comme par exemple la silice pyrogénée.

Cette ou ces charges peuvent être présentes à raison de 0,1 à 20 % en poids, de préférence 2 à 15 % en poids, et mieux de 2 % à 10 % en poids par rapport au poids total de la composition de type fond de teint, notamment des compositions de base ou de surface du produit de type fond de teint.

### Milieu physiologiquement acceptable

Par « milieu physiologiquement acceptable », on désigne un milieu non toxique et susceptible d'être appliqué sur la peau d'êtres humains. Le milieu physiologiquement acceptable est généralement adapté à la nature de la peau sur laquelle doit être appliquée la composition de type fond de teint ainsi qu'à la forme sous laquelle la composition est destinée à être conditionnée, notamment fluide à la température ambiante et sous pression atmosphérique.

Comme précisé précédemment, les composition de type fond de teint appliquées selon l'invention, notamment les compositions de base et/ou de surface du produit de type fond de teint selon l'invention, peuvent être formulées sous une forme fluide ou solide de type poudre, libre, compacte ou coulée.

Elles peuvent notamment, indépendamment l'une de l'autre, se présenter sous une forme anhydre ou sous la forme d'un gel, d'émulsion directe, inverse ou multiple associant au moins une phase aqueuse et au moins une phase grasse.

### Phase aqueuse

La composition de type fond de teint appliquée selon l'invention, notamment la composition de base et/ou la composition de surface du produit de type fond de teint selon l'invention, peut comprendre au moins un milieu aqueux, constituant une phase aqueuse, qui peut former la phase continue de la composition de type fond de teint considérée.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 %, en poids par rapport au poids total de la composition considérée.

Un tel milieu peut comprendre en outre une huile volatile telle que définie ci-après.

### Phase grasse

La composition de type fond de teint appliquée selon l'invention, notamment la composition de base et/ou la composition de surface du produit de type fond de teint selon l'invention, peut comporter une phase grasse et notamment au moins un corps gras liquide à température ambiante (25°C) et/ou un corps gras solide à température ambiante tel que les cires, les corps gras pâteux, les gommes et leurs mélanges. La phase grasse peut en outre contenir des solvants organiques lipophiles.

La composition de type fond de teint peut posséder par exemple une phase grasse continue, pouvant contenir moins de 5% d'eau, notamment moins de 1% d'eau par rapport à son poids total et en particulier être sous forme anhydre.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées, des huiles siliconées, ou leurs mélanges. On entend par "huile hydrocarbonée", une huile contenant principalement des atomes d'hydrogène et de carbone et éventuellement des atomes d'oxygène, d'azote, de soufre, de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permetyls^{®}, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt^{®} par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans une composition selon l'invention à une teneur allant de 0,1 à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées fluorées et/ou siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810, 812 et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

Les huiles non volatiles peuvent être présentes dans une composition selon l'invention en une teneur allant de 0,01 à 90 % en poids, notamment de 0,1 à 85 % en poids, et en particulier de 1 % à 70 % en poids, par rapport au poids total de la composition.

Plus généralement, le corps gras liquide peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Ainsi, une composition appliquée selon l'invention peut comprendre au moins un composé gras pâteux à température ambiante.

Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition de type fond de teint selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges. Comme triglycéride d'origine végétale, on peut utiliser les dérivés d'huile de ricin hydrogénée, tels que le « THIXINR » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Coming sous les noms commerciaux de DC2503^{®} et DC25514^{®}, et leurs mélanges.

Le corps gras pâteux peut être présent dans une composition selon l'invention en une teneur allant de 0,01 à 50% en poids, de préférence allant de 0,1 à 45 % en poids, et mieux allant de 0,2 % à 30 % en poids, par rapport au poids total de ladite composition.

La composition de type fond de teint appliquée selon l'invention, notamment la composition de base et/ou la composition de surface du produit de type fond de teint selon l'invention, peut comprendre en outre une cire. La cire peut être solide à température ambiante (25°C), à changement d'état solide/liquide réversible, ayant une température de fusion supérieure à 30°C pouvant aller jusqu'à 200°C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, les cires microcristallines, et leurs mélanges.

En particulier, la cire peut être présente sous forme d'émulsion cire-dans-eau.

La cire peut être présente dans une composition selon l'invention en une teneur allant de 0,01 % à 50 % en poids, en particulier de 0,1 % à 30 % en poids, et notamment de 0,2 % à 20 % en poids, par rapport au poids total de la composition.

### Agents tensioactifs

La composition de type fond de teint appliquée selon l'invention, notamment la composition de base et/ou la composition de surface du produit de type fond de teint selon l'invention, peut en outre contenir des agents tensioactifs émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition de type fond de teint selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

### Polymère filmogène

La composition de type fond de teint appliquée selon l'invention, notamment la composition de base et/ou la composition de surface du produit de type fond de teint selon l'invention, peut comprendre, en outre, au moins un polymère filmogène.

Dans la présente demande, on entend par « polymère filmogène », un polymère apte à former à lui seul ou en présence d'un agent auxiliaire de filmification, un film continu et adhérent sur la peau.

On utilise de préférence un polymère filmogène apte à former un film hydrophobe, c'est-à-dire un polymère dont le film a une solubilité dans l'eau à 25 °C inférieure à 1 % en poids.

Le polymère filmogène peut notamment être au moins un polymère choisi parmi le groupe comprenant :
- les polymères filmogènes hydrosolubles,
- des dispersions aqueuses de particules de polymères filmogènes hydrodispersibles, encore appelées « latex » ; dans ce cas, la composition de type fond de teint doit comprendre une phase aqueuse,
- des polymères filmogènes liposolubles,
- les polymères filmogènes lipodispersibles sous forme de dispersions non aqueuses de particules de polymère, de préférence des dispersions de particules polymériques, le cas échéant stabilisées en leur surface par au moins un agent stabilisant, dans une ou plusieurs huiles de silicones et/ou hydrocarbonées ; ces dispersions non aqueuses sont encore appelées « NAD ».

La composition de type fond de teint peut comprendre parallèlement un mélange de ces polymères.

Le polymère filmogène peut être présent dans une composition selon l'invention en une teneur en matières sèches allant de 0,01 % à 20 % en poids par rapport au poids total de la composition et notamment de 0,5 % à 10 % en poids.

Parmi les polymères filmogènes utilisables selon l'invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C1-C20, des (méth)acrylates d'aryle, en particulier d'aryle en C₆-C₁₀, des (méth)acrylates d'hydroxyalkyle, en particulier d'hydroxyalkyle en C₂-C₆.

Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.

Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C2-C12. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.

Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Dans un premier exemple de réalisation de la composition de type fond de teint selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEY KOGYO; ou ben encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®} , SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Dans un second exemple de réalisation de la composition de type fond de teint selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   - les gommes arabiques, la gomme de guar, les dérivés du xanthane, la gomme de karaya ;
   - les alginates et les carraghénanes ;
   - les glycoaminoglycanes, l'acide hyaluronique et ses dérivés ;
   - la résine shellac, la gomme de sandaraque, les dammars, les élémis, les copals ;
   - l'acide désoxyribonucléïque ;
   - les muccopolysaccharides tels que l'acide hyaluronique, les chondroïtines sulfate, et leurs mélanges.

Selon une autre variante de réalisation de la composition de type fond de teint selon l'invention, le polymère filmogène peut être présent dans une phase grasse liquide comprenant des huiles ou solvants organiques tels que ceux décrits précédemment. Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.

De préférence, la phase grasse liquide comprend une huile volatile, éventuellement en mélange avec une huile non volatile, les huiles pouvant être choisies parmi les huiles citées précédemment.

Dans un troisième exemple de réalisation de la composition de type fond de teint selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747.

Les particules de polymère sont stabilisées en surface grâce à un stabilisant qui peut être un polymère séquencé, un polymère greffé, et/ou un polymère statistique, seul ou en mélange.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Dans un quatrième exemple de réalisation de la composition de type fond de teint selon l'invention, le polymère filmogène peut être solubilisé dans la phase grasse liquide, on dit alors que le polymère filmogène est un polymère liposoluble.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui ont pour but qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition appliquée selon invention peut en outre comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

La composition ou le produit de type fond de teint appliqués selon l'invention se présente généralement sous la forme d'un fond de teint notamment à appliquer sur le visage ou le cou, d'un produit anti-cernes, d'un correcteur de teint, d'une crème teintée ou base de maquillage pour le visage ou d'une composition de maquillage pour le corps.

La composition de type fond de teint appliquée selon l'invention, notamment la composition de base et/ou la composition de surface du produit de type fond de teint selon l'invention, peut se présenter sous une forme solide, par exemple pulvérulente, compactée ou coulée ou sous forme stick ou sous la forme d'un fluide par exemple pâteux ou liquide. Elle peut aussi se présenter sous forme de pâte souple, d'un onguent, d'une pommade solide ou fluide de type crème. Par exemple, elle peut être une émulsion huile-dans-eau ou eau-dans-huile, un gel notamment anhydre, solide ou souple et même sous forme biphasique. Selon cette variante, elle se présente plus particulièrement sous forme d'un fond de teint à phase continue huileuse et notamment anhydre ; dans ce cas, elle peut contenir une phase aqueuse à un taux inférieur à 5 %.

Dans le cas du produit appliqué selon l'invention, les deux compositions correspondantes peuvent se présenter sous des formes identiques ou différentes et, notamment conformes à ce qui précède.

La composition de type fond de teint appliquée selon l'invention, notamment la composition de base et/ou la composition de surface du produit de type fond de teint selon l'invention, peut être fabriquée par les procédés connus, généralement utilisés dans le domaine cosmétique.

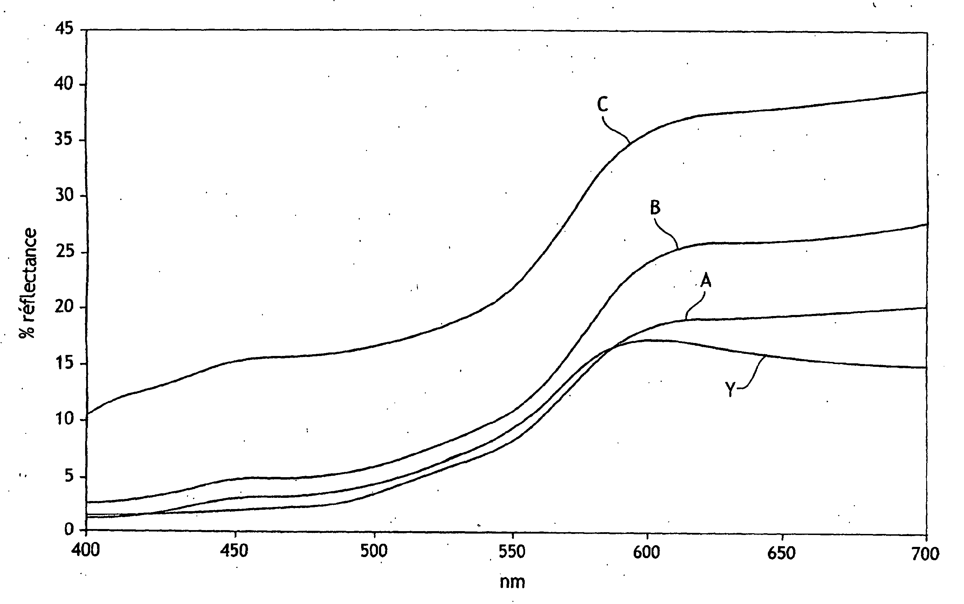

L'invention pourra être mieux comprise à la lecture de la description détaillée qui va suivre, d'exemples de mise en oeuvre non limitatifs de celle-ci et de la figure 1 :
Figure 1 : représentation des spectres de réflectance des compositions A, B, C et Y.

On a préparé 12 compositions de fond de teint comme suit, les proportions y sont exprimées en pourcentages massiques :

### Composition A

Il s'agit d'un fond de teint plus particulièrement destiné aux peaux foncées, sa formulation est comme suit :
- polyméthylcétyldiméthylméthylsiloxane oxyéthyléné (ABIL EM 90 de la société GOLDSCHMIDT) 0,80 %
- iso-stéarate de polyglycérol (4 moles) (ISOLAN GI 34 de la société GOLDSCHMIDT) 0,60 %
- laurate d'hexyle 0,60 %
- polydiméthylsiloxane oxyéthyléné (DP : 70 - Viscosité : 500 CST) (KF-6017 de la société SHIN ETSU SILICONES) 4,48 %
- isoéicosane (PERMETHYL 102 A de PERMETHYL) 2 %
- polydiméthylsiloxane (viscosité : 5 CST) (FLUID 200 5 CS de la société DOW CORNING) 2 %
- néopentanoate d'iso-stéaryle 0,50 %
- cyclohéxadiméthylsiloxane 8 %
- cyclopentadiméthylsiloxane 11,36 %
- iso-dodécane 13 %
- D,L-alpha-tocophérol (VITAMINE E) 0,08 %
- hectorite modifiée par du chlorure de distéaryl di-méthyl ammonium (BENTONE 38V de la société ELEMENTIS) 1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 1,86 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO) 0,72 %
- oxyde de fer noir enrobé de phosphate de perfluoroalkyle en dispersion à 65 % en poids dans cyclométhicone/diméthyl polysiloxane copolyol (CI : 77499) (FA65DBF de la société KOBO) 0,34 %
- oxyde de titane traité alumine enrobé de phosphate de perfluoroalkyle en dispersion à 65 % en poids dans décaméthylcyclopentasiloxane / diméthiconecopolyol (CI 77891) (FA65DF de la société KOBO) 7,39 %
- microsphères creuses de polyméthacrylate de méthyle (granulométrie : 10 à 12 microns), (COVABEAD LH 85 de la société WACKHERR) 4 %
- butylèneglycol-1,3 10 %
- chlorure de sodium 0,70 %
- conservateurs 0,90 %
- mica-oxyde de fer brun (60/40) (CI : 77019 + 77491) (COLORONA PASSION ORANGE de la société MERCK) 2 %
- sel di-sodique de tartrazine (CI : 19140) (FD & C YELLOW 5 de la société LCW) 2 %
- eau quantité suffisante pour (qsp) 100 %

### Composition B

Il s'agit d'un fond de teint convenant plus particulièrement aux peaux foncées claires, sa formulation est la suivante :
- polyméthylcétyldiméthylméthylsiloxane oxyéthyléné (ABIL EM 90 de la société GOLDSCHMIDT) 0,80 %
- iso-stéarate de polyglycérol (4 moles) (ISOLAN GI 34 de la société GOLDSCHMIDT) 0,60 %
- laurate d'hexyle 0,60 %
- polydiméthylsiloxane oxyéthyléné (DP : 70 - Viscosité : 500 CST) (KF-6017 de la société SHIN ETSU SILICONES) 4,48 %
- isoéicosane (PERMETHYL 102A de la société PERMETHYL) 2 %
- polydiméthylsiloxane (viscosité : 5 CST) (FLUID 200 5 CS de la société DOW CORNING) 2 %
- néopentanoate d'iso-stéaryle 0,50 %
- cyclohéxadiméthylsiloxane 8 %
- cyclopentadiméthylsiloxane 11,36 %
- iso-dodécane 13 %
- D,L-alpha-tocophérol (VITAMINE E) 0,08 %
- hectorite modifiée par du chlorure de distéaryl di-méthyl ammonium (BENTONE 38V de la société ELEMENTIS) 1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 6,90 %
- mica-oxychlorure de bismuth-oxyde de fer brun (47/28/25) (CHROMA - LITE BROWN de la société ENGELHARD) 6,81 %
- laque d'aluminium du rouge Allura sur alumine (40/60) (FD&C Red 40 Al lake de la société NOVEON) 0,60 %
- microsphères creuses de polyméthacrylate de méthyle (granulométrie : 10 à 12 microns), (COVABEAD LH 85 de la société WACKHERR) 4 %
- butylèneglycol-1,3 10 %
- chlorure de sodium 0,70 %
- conservateurs 0,90 %
- eau qsp 100 %

### Composition C

Cette composition convient tout particulièrement aux peaux noires, sa formulation est la suivante :
- polyméthylcétyldiméthylméthylsiloxane oxyéthylène (ABIL EM 90 de la société GOLDSCHMIDT) 0,80 %
- iso-stéarate de polyglycérol (4 moles) (ISOLAN GI 34 de la société GOLDSCHMIDT) 0,60 %
- laurate d'hexyle 0,60 %
- polydiméthylsiloxane oxyéthyléné (DP : 70 - Viscosité : 500 CST) (KF-6017 de la société SHIN ETSU SILICONES) 4,48 %
- isoéicosane (PERMETHYL 102A) 2 %
- polydiméthylsiloxane (viscosité : 5 CST) (FLUID 200 5 CS de la société DOW CORNING) 2 %
- néopentanoate d'iso-stéaryle 0,50 %
- cyclohéxadiméthylsiloxane 8 %
- cyclopentadiméthylsiloxane 11,36 %
- iso-dodécane 13 %
- D,L-alpha-tocophérol (VITAMINE E) 0,08 %
- hectorite modifiée par du chlorure de distéaryl di-méthyl ammonium (BENTONE 38V de la société ELEMENTIS) 1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 1,86 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO) 0,72 %
- oxyde de fer noir enrobé de phosphate de perfluoroalkyle en dispersion à 65 % en poids dans cyclométhicone/diméthyl polysiloxane copolyol (CI : 77499) (FA65DBF de la société KOBO) 0,34 %
- oxyde de titane traité alumine enrobé de phosphate de perfluoroalkyle en dispersion à 65 % en poids dans décaméthylcyclopentasiloxane/diméthiconecopolyol (CI 77891) (FA65DF de la société KOBO) 7,39 %
- microsphères creuses de polyméthacrylate de méthyle (granulométrie : 10 à 12 microns), (COVABEAD LH 85 de la société WACKHERR) 4 %
- butylèneglycol-1,3 10 %
- chlorure de sodium 0,70 %
- conservateurs 0,90 %
- silice-oxyde de fer brun (XIRONA INDIAN SUMMER de MERCK) 4 %
- eau qsp 100 %

### Composition D

Il s'agit d'une composition fond de teint convenant plus particulièrement aux peaux noires, sa formulation est la suivante :
- polyméthylcétyldiméthylméthylsiloxane oxyéthyléné (ABIL EM 90 de la société GOLDSCHMIDT) 0,80 %
- iso-stéarate de polyglycérol (4 moles) (ISOLAN GI 34 de la société GOLDSCHMIDT) 0,60 %
- laurate d'hexyle 0,60 %
- polydiméthylsiloxane oxyéthyléné (DP : 70 - Viscosité : 500 CST) (KF-6017 de la société SHIN ETSU SILICONES) 4,48 %
- isoéicosane (PERMETHYL 102A) 2 %
- polydiméthylsiloxane (viscosité : 5 CST) (FLUID 200 5 CS de la société DOW CORNING) 2 %
- néopentanoate d'iso-stéaryle 0,50 %
- cyclohéxadiméthylsiloxane 8 %
- cyclopentadiméthylsiloxane 11,36 %
- iso-dodécane 13 %
- D,L-alpha-tocophérol (VITAMINE E) 0,08 %
- hectorite modifiée par du chlorure de distéaryl di-méthyl ammonium (BENTONE 38V de la société ELEMENTIS) 1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 5,25 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO) 0,72 %
- oxyde de fer noir enrobé de phosphate de perfluoroalkyle en dispersion à 65 % en poids dans cyclométhicone/diméthyl polysiloxane copolyol (CI : 77491) (FA65DBF de la société KOBO) 0,34 %
- oxyde de titane traité alumine enrobé de phosphate de perfluoroalkyle en dispersion à 65 % en poids dans décaméthylcyclopentasiloxane/diméthiconecopolyol (CI 77891) (FA65DF de la société KOBO) 4 %
- microsphères creuses de polyméthacrylate de méthyle (granulométrie : 10 à 12 microns), (COVABLEAD LH 85 de la société WACKHERR) 4 %
- butylèneglycol-1,3 10 %
- chlorure de sodium 0,70 %
- silice-oxyde de fer brun (XIRONA INDIAN SUMMER de la société MERCK) 4 %
- conservateurs 0,90 %
- eau qsp 100 %

### Composition E

Il s'agit d'un stick pour peau noire, sa formulation est la suivante :
- cire de polyéthylène (PM : 500) (Polywax 500 de BARECO) 4 %
- homopolymère de l'éthylène (Point de fusion : 79,5 °C) (PERFORMALENE 400 de NEW PHASE TECHNOLOGIE) 8 %
- cyclopentadiméthylsiloxane 5 %
- cyclohéxadiméthylsiloxane 20 %
- iso-dodécane 19 %
- phényl triméthylsiloxy trisiloxane (viscosité : 20 CST - PM : 372) (DC556 de DOW CORNING) 19 %
- microsphères creuses de polyméthacrylate de méthyle (granulomètrie : 10 à 12 microns) (COVABLAD LH85 de WACKHERR) 10 %
- mica-oxyde de fer brun (94/6) (CI : 77019 + 77491) (COSMETICA ORANGE de ENGELHARD) 12 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 3 %

### Composition F

Il s'agit d'un fond de teint fluide pour peaux noires. Sa formulation est comme suit :
- cétyl polyéthylène glycol/PPG - 10/1 diméthicone (ABIL EM 90) 0,80 %
- 4-isostérate de polyglycéryle (ISOLAN GI34 de la société GOLDSCHMIDT) 0,60 %
- laurate d'hexyle 0,60 %
- PEG-10 diméthicone (KF6017) 4,48 %
- isoéicosane (PERMETHYL 102A) 2 %
- diméthicone (DC 200 FLUID) 2 %
- néopentanoate d'isostearyle 0,50 %
- cyclohexasiloxane 8 %
- cyclopentasiloxane 11,36 %
- isododécane 13 %
- D, L-alpha-tocophérol (VITAMINE E) 0,08 %
- hectorite modifiée par du chlorure de distéaryl di-méthyl ammonium (BENTONE 38V de la société ELEMENTIS) 1,6 %
- oxydes de fer et cyclopentasiloxane et PEG/PPG-18/18 diméthicone et phosphates de fluoroalcool en C₉ à C₁₅ (FA5ODYF de KOBO) 4,73 %
- oxydes de fer et cyclopentasiloxane et PEG/PPG-18/18 diméthicone et phosphates de fluoroalcool en C₉ à C₁₅ (FA50DRF de KOBO) 2,595 %
- oxydes de fer et cyclopentasiloxane et PEG/PPG-18/18 diméthicone et phosphates de fluoroalcool en C₉ à C₁₅ (FA65DBF de KOBO) 1,22 %
- dioxyde de titane et cyclopentasiloxane et PEG/PPG-18/18 diméthicone et phosphates de fluoroalcool en C₉ à C₁₅ et alumine (FA65DF de KOBO) 1,765 %
- polyméthacrylate de méthyl (COVABEAD LH 85) 4 %
- butylène glycol 10 %
- chlorure de sodium 0,70 %
- conservateurs 0,90 %
- mica et oxydes de fer (COLORONA PASSION ORANGE de MERCK) 2 %
- lake yellow 6 (pigments : FDC YELLOW 6 A1 LAKE DE SUN CHEMICAL) (CI15985) 2 %
- eau qsp 100 %

### Composition Y

Il s'agit d'un fond de teint plus particulièrement destiné aux peaux foncées, sa formulation est comme suit :
- polyméthylcétyldiméthylméthylsiloxane oxyéthyléné (ABIL EM 90 de la société GOLDSCHMIDT) 0,80 %
- iso-stéarate de polyglycérol (4 moles) (ISOLAN GI 34 de la société GOLDSCHMIDT) 0,60 %
- laurate d'hexyle 0,60 %
- polydiméthylsiloxane oxyéthyléné (DP : 70 - Viscosité : 500 CST) (KF-6017 de la société SHIN ETSU SILICONES) 4,48 %
- isoeicosane PERMETHYL 102 A de PERMETHYL 2 %
- polydiméthylsiloxane (viscosité : 5 CST) (FLUID 200 5 CST de la société DOW CORNING) 2 %
- néopentanoate d'iso-stéaryle 0,5 %
- cyclohéxadiméthylsiloxane 8 %
- cyclopentadiméthylsiloxane 11,3 %
- iso-dodécane 13 %
- D,L-alpha-tocophérol (VITAMINE E) 0,08 %
- hectorite modifiée par du chlorure de distéaryl di-méthyl ammonium (BENTONE 38V de la société ELEMENTIS) 1,60 %
- oxyde de fer jaune enrobé de phosphate de perfluoroalkyle en dispersion à 50 % en poids dans décaméthylcyclopentasiloxane/diméthicone copolyol (CI : 77492) (FA50DYF de la société KOBO) 10,93 %
- oxyde de fer brun enrobé de phosphate de perfluoroalkyle en dispersion à 50 % en poids dans cyclométhicone/diméthyl polysiloxane copolyol (CI : 77491) (FA50DRF de la société KOBO) 2,15 %
- microsphères creuses de polyméthacrylate de méthyle (granulométrie : 10 à 12 microns), (COVABEAD LH 85 de la société WACKHERR) 4 %
- mica-bleu ferrique vendu sous la dénomination MICRONA MATTE BLUE par la société MERCK 1,23 %
- butylèneglycol-1,3 10 %
- chlorure de sodium 0,70 %
- conservateurs 0,90 %
- eau qsp 100 %

### Composition G

- polymethylcétyldiméthylméthylsiloxane oxyethyléné (ABIL EM 90 de la société GOLDSCHMIDT) 0,80 %
- iso-stéarate de polyglycérol (4 moles) (ISOLAN GI34 de la société GOLDSCHMIDT) 0,60 %
- laurate d'hexyle 0,60 %
- polydiméthylsiloxane oxyethyléné (DP : 70 - Viscosité : 500 CST) (KF-6017 de la société SHIN ETSU SILICONES) 4,48 %
- isoéicosane (PERMETHYL 102A de PERMETHYL) 2 %
- polydimethylsiloxane (viscosité 5 CST) (FLUID 200 5 CS de la société DOW CORNING) 2 %
- néopentanoate d'iso-stéaryle 0,5 %
- cyclohexadimethylsiloxane 8 %
- isododécane 13 %
- D,L-alpha-tocophérol (VITAMINE E) 0,08 %
- Smectite: silicate de magnésium modifié dans cyclopentadiméthylsiloxane et éthanol (GEL DE BENTONE VS 5 V) 12,96 %
- Oxyde de fer jaune enrobé de phosphate de perfluoroalkyle (PF 5 YELLOW 601 de chez DAITO) 1,28 %
- Oxyde de fer brun enrobé de phosphate de perfluoroalkyle (PF 5 RED R 516 L de chez DAITO) 2,13 %
- Oxyde de fer noir enrobé de phosphate de perfluoroalkyle (PF 5 BLACK BL 100 de chez DAITO) 0,47 %
- Bleu d'outremer enrobé de phosphate de perfluoroalkyle (PF 5 ULTRAMARINE N° 801 de chez DAITO) 1,28 %
- cyclopentadimethylsiloxane (DOW CORNING 245 FLUID de chez DOW CORNING) 5,17 %
- microsphères creuses de polymethacrylate de methyle (granulométrie 10 à 12 µm), (COVABEAD LH85 de la société WACKHERR) 4 %
- butylèneglycol-1,3 10 %
- chlorure de sodium 0,7 %
- conservateurs 0,90 %
- phenoxyethanol (PHENOXETOL) 0,5 %
- mica-oxyde de fer brun (60/40) (CI : 77019+77491) (COLORONA PASSION ORANGE de la société MERCK) 2 %
- lake yellow 6 (pigments : FDC YELLOW 6 A1 LAKE DE SUN CHEMICALS) (CI1 5985) 2 %
- eau qsp 100 %

### Composition H

- polymethylcétyldiméthylméthylsiloxane oxyethyléné (ABIL EM 90 de la société GOLDSCHMIDT) 0,80 %
- iso-stéarate de polyglycérol (4 moles) (ISOLAN GI34 de la société GOLDSCHMIDT) 0,60 %
- laurate d'hexyle 0,60 %
- polydiméthylsiloxane oxyethyléné (DP : 70 - Viscosité : 500 CST) (KF-6017 de la société SHIN ETSU SILICONES) 4,48 %
- isoéicosane (PERMETHYL 102A de PERMETHYL) 2 %
- polydimethylsiloxane (viscosité 5 CST) (FLUID 200 5 CS de la société DOW CORNING) 2 %
- néopentanoate d'iso-stéaryle 0,5 %
- cyclohexadimethylsiloxane 8 %
- isododécane 13 %
- D,L-alpha-tocophérol (VITAMINE E) 0,08 %
- Smectite: silicate de magnésium modifié dans cyclopentadiméthylsiloxane et éthanol (GEL DE BENTONE VS 5 V) 12,96 %
- Oxyde de fer jaune enrobé de phosphate de perfluoroalkyle (PF 5 YELLOW 601 de chez DAITO) 2,37 %
- Oxyde de fer brun enrobé de phosphate de perfluoroalkyle (PF 5 RED R 516 L de chez DAITO) 1,3 %
- Oxyde de fer noir enrobé de phosphate de perfluoroalkyle (PF 5 BLACK BL 100 de chez DAITO) 0,61 %
- Oxyde de titane enrobé de phosphate de perfluoroalkyle (PF 5 ULTRAMARINE N° 801 de chez DAITO) 0,88%
- cyclopentadimethylsiloxane (DOW CORNING 245 FLUID de chez DOW CORNING) 5,13 %
- microsphères creuses de polymethacrylate de methyle (granulométrie 10 à 12 µm), (COVABEAD LH85 de la société WACKHERR) 4 %
- butylèneglycol-1,3 10 %
- chlorure de sodium 0,7 %
- conservateurs 0,90 %
- phenoxyethanol (PHENOXETOL) 0,5 %
- mica-oxyde de fer brun (60/40) (CI : 77019+77491) (COLORONA PASSION ORANGE de la société MERCK) 2 %
- lake yellow 6 (pigments : FDC YELLOW 6 A1 LAKE DE SUN CHEMICALS) (CI15985) 2 %
- eau qsp 100 %

### Composition I

- polymethylcétyldiméthylméthylsiloxane oxyethyléné (ABIL EM 90 de la société GOLDSCHMIDT) 0,80 %
- iso-stéarate de polyglycérol (4 moles) (ISOLAN GI34 de la société GOLDSCHMIDT) 0,60 %
- laurate d'hexyle 0,60 %
- polydiméthylsiloxane oxyethyléné (DP : 70 - Viscosité : 500 CST) (KF-6017 de la société SHIN ETSU SILICONES) 4,48 %
- isoéicosane (PERMETHYL 102A de PERMETHYL) 2 %
- polydimethylsiloxane (viscosité 5 CST) (FLUID 200 5 CS de la société DOW CORNING) 2 %
- néopentanoate d'iso-stéaryle 0,5 %
- cyclohexadimethylsiloxane 8 %
- isododécane 13 %
- D,L-alpha-tocophérol (VITAMINE E) 0,08 %
- Smectite: silicate de magnésium modifié dans cyclopentadimethylsiloxane et éthanol (GEL DE BENTONE VS 5 V) 12,96 %
- Oxyde de fer jaune enrobé de phosphate de perfluoroalkyle (PF 5 YELLOW 601 de chez DAITO) 3,46 %
- cyclopentadimethylsiloxane (DOW CORNING 245 FLUID de chez DOW CORNING) 3,46 %
- microsphères creuses de polymethacrylate de methyle (granulométrie 10 à 12 µm), (COVABEAD LH85 de la société WACKHERR) 4 %
- butylèneglycol-1,3 10 %
- chlorure de sodium 0,7 %
- conservateurs 0,90 %
- phenoxyethanol (PHENOXETOL) 0,5 %
- Mica-oxychlorure de bismuth-oxyde de fer brun (CHROMA-LITE BROWN 4509 de chez ENGELHARD) 6,81 %
- Laque d'aluminium du rouge allura sur alumine (FDC RED 40 AL LAKE - 6808 de chez NOVEON) 0,6 %
- eau qsp 100%

### Composition J

- polymethylcétyldiméthylméthylsiloxane oxyethyléné (ABIL EM 90 de la société GOLDSCHMIDT) 0,80 %
- iso-stéarate de polyglycérol (4 moles) (ISOLAN GI34 de la société GOLDSCHMIDT) 0,60 %
- laurate d'hexyle 0,60 %
- polydiméthylsiloxane oxyethyléné (DP : 70 - Viscosité : 500 CST) (KF-6017 de la société SHIN ETSU SILICONES) 4,48 %
- isoéicosane (PERMETHYL 102A de PERMETHYL) 2 %
- polydimethylsiloxane (viscosité 5 CST) (FLUID 200 5 CS de la société DOW CORNING) 2 %
- néopentanoate d'iso-stéaryle 0,5 %
- cyclohexadimethylsiloxane 8 %
- isododécane 13 %
- D,L-alpha-tocophérol (VITAMINE E) 0,08 %
- Smectite: silicate de magnésium modifié dans cyclopentadiméthylsiloxane et éthanol (GEL DE BENTONE VS 5 V) 12,96 %
- Oxyde de fer jaune enrobé de phosphate de perfluoroalkyle (PF 5 YELLOW 601 de chez DAITO) 1,75 %
- Oxyde de fer brun enrobé de phosphate de perfluoroalkyle (PF 5 RED R 516 L de chez DAITO) 0,39%
- Oxyde de fer noir enrobé de phosphate de perfluoroalkyle (PF 5 BLACK BL 100 de chez DAITO) 0,14 %
- Oxyde de titane enrobé de phosphate de perfluoroalkyle (PF 5 TIO2 A 100 de chez DAITO) 2,22 %
- cyclopentadimethylsiloxane (DOW CORNING 245 FLUID de chez DOW CORNING) 4,49 %
- microsphères creuses de polymethacrylate de methyle (granulométrie 10 à 12 µm), (COVABEAD LH85 de la société WACKHERR) 4 %
- butylèneglycol-1,3 10 %
- chlorure de sodium 0,7 %
- conservateurs 0,90 %
- phenoxyethanol (PHENOXETOL) 0,5 %
- Silice-oxyde de fer brun (XIRONA INDIAN SUMMER de chez MERCK) 3 %
- Mica-oxychlorure de bismuth-oxyde de fer jaune (CHROMA-LITE YELLOW 4502 de chez ENGELHARD) 2,32 %
- eau qsp 100 %

### Composition K

- polymethylcétyldiméthylméthylsiloxane oxyethyléné (ABIL EM 90 de la société GOLDSCHMIDT) 0,80 %
- iso-stéarate de polyglycérol (4 moles) (ISOLAN GI34 de la société GOLDSCHMIDT) 0,60 %
- laurate d'hexyle 0,60 %
- polydiméthylsiloxane oxyethyléné (DP : 70 - Viscosité : 500 CST) (KF-6017 de la société SHIN ETSU SILICONES) 4,48 %
- isoéicosane (PERMETHYL 102A de PERMETHYL) 2 %
- polydimethylsiloxane (viscosité 5 CST) (FLUID 200 5 CS de la société DOW CORNING) 2 %
- néopentanoate d'iso-stéaryle 0,5 %
- cyclohexadimethylsiloxane 8 %
- isododécane 13 %
- D,L-alpha-tocophérol (VITAMINE E) 0,08 %
- Smectite: silicate de magnésium modifié dans cyclopentadiméthylsiloxane et éthanol (GEL DE BENTONE VS 5 V) 12,96 %
- Oxyde de fer jaune enrobé de phosphate de perfluoroalkyle (PF 5 YELLOW 601 de chez DAITO) 1,75 %
- Oxyde de fer brun enrobé de phosphate de perfluoroalkyle (PF 5 RED R 516 L de chez DAITO) 0,39 %
- Oxyde de fer noir enrobé de phosphate de perfluoroalkyle (PF 5 BLACK BL 100 de chez DAITO) 0,19 %
- Oxyde de titane enrobé de phosphate de perfluoroalkyle (PF 5 TIO2 A 100 de chez DAITO) 2,17 %
- cyclopentadimethylsiloxane (DOW CORNING 245 FLUID de chez DOW CORNING) 4,49 %
- microsphères creuses de polymethacrylate de methyle (granulométrie 10 à 12 µm), (COVABEAD LH85 de la société WACKHERR) 4 %
- butylèneglycol-1,3 10 %
- chlorure de sodium 0,7 %
- conservateurs 0,90 %
- phenoxyethanol (PHENOXETOL) 0,5 %
- Silice-oxyde de fer brun (XIRONA INDIAN SUMMER de chez MERCK) 3,5 %
- Mica-oxychlorure de bismuth-oxyde de fer jaune (CHROMA-LITE YELLOW 4502 de chez ENGELHARD) 1,83 %
- eau qsp 100 %

### TEST DE REFLECTANCE

On a maquillé avec des compositions A, B, C et Y des peaux foncées.

La couleur de chaque composition A, B, C, Y, G, H, I, J et K est donnée dans le tableau suivant, les valeurs étant indiquées avec une précision de 15 %, mieux 10 %, mieux encore 5 % :

| Composition | L* | a* | b* | C* | h(°) |
|---|---|---|---|---|---|
| A | 39,46 | 18,63 | 37,11 | 41,52 | 63,34 |
| B | 48,59 | 20,21 | 31,91 | 37,77 | 57,65 |
| C | 57,24 | 14,69 | 19,65 | 24,53 | 53,22 |
| Y | 39,22 | 13,80 | 32,25 | 35,08 | 66,83 |
| G | 38,04 | 17,12 | 15,45 | 23,06 | 42,08 |
| H | 43,50 | 20,25 | 21,19 | 29,31 | 46,30 |
| I | 48,38 | 18,73 | 23,51 | 30,06 | 51,46 |
| J | 55,89 | 17,55 | 23,30 | 29,17 | 53 |
| K | 55,78 | 16,85 | 21,51 | 27,32 | 51,93 |

Pour mesurer la couleur d'une composition, on a procédé à une mesure de couleur dans la masse du produit, comme suit.

On a rempli une cuve référencée H247 avec la composition. On a arasé puis appliqué sur la composition une lame de verre en prenant soin d'éviter les bulles d'air sous la lame. On a effectué une mesure de couleur avec un spectrocolorimètre de marque MINOLTA^{®} de type CM-2002 en moyenne ouverture et en mode réflexion spéculaire inclus.

On a également effectué une mesure de réflectance spectrale, dans la masse, à l'aide d'un spectrocolorimètre de marque MINOLTA^{®} de type CM3700d en mode réflexion, spéculaire exclu, UV inclus et avec une petite ouverture d/8.

La figure 1 représente les spectres de réflectance des compositions A, B, C et Y présentes dans les mêmes coupelles que ci-dessus.

On remarque que pour la région de longueurs d'onde comprise entre 600 et 680 nm, la réflectance est comprise entre 10 et 45 %, plus précisément entre 12 et 40 %, pour les quatre compositions testées.

La réflectance est inférieure à 20 % dans l'intervalle allant de 450 à 500 nm.

## Revendications

1. Procédé de maquillage d'une peau dont la clarté moyenne L* mesurée sur le front, les pommettes et le menton dans l'espace colorimétrique CIE 1976, est inférieure à 60, **caractérisé en ce qu'**il comprend l'application sur ladite peau d'au moins une composition de type fond de teint associant, dans un milieu physiologiquement acceptable, au moins un agent de coloration possédant une réfléctance significative d'une coloration jaune ou orangée dans l'intervalle 550 à 675 nm et au moins une nacre à titre de particules réfléchissantes, cette association étant apte à conférer à ladite composition, un angle de teinte h variant de 40° à 70°, notamment de 50° à 70° et une saturation C* variant de 20 à 50.

2. Procédé selon la revendication 1, **caractérisé en ce que** ladite composition comprend de 0,5 à 30 %, notamment de 2 à 20 % et en particulier de 5 à 18 % en poids d'agent(s) de coloration par rapport à son poids total.

3. Procédé selon l'une quelconque des revendications précédentes, **caractérisé en ce que** ladite composition comprend de 0,5 à 60 % en poids, notamment de 1 à 30 % en poids, en particulier de 2 à 20 % de particules réfléchissantes et plus particulièrement de 3 à 10 % en poids, par rapport à son poids total.

4. Procédé selon l'une quelconque des revendications précédentes, **caractérisé en ce que** les particules réfléchissantes possèdent une couleur ou un reflet jaune, rose, rouge, bronze, orangé, brun, or, et/ou cuivré.

5. Procédé selon l'une quelconque des revendications précédentes, **caractérisé en ce que** ledit agent de coloration est choisi parmi les pigments minéraux ou organiques, les polymères colorants, les colorants hydrosolubles ou liposolubles, les laques organiques, les poudres métalliques et leurs mélanges.

6. Procédé selon la revendication 5, **caractérisé en ce que** les pigments minéraux sont choisis parmi les oxydes métalliques jaunes, rouges, bruns et notamment les oxydes de fer.

7. Procédé selon la revendication 5 ou 6, **caractérisé en ce que** le colorant hydrosoluble est choisi parmi le colorant brun identifié par l'appellation « caramel » selon le Color Index ; les colorants jaunes identifiés par les numéros de Color Index n° 10316, 13015, 18690, 18820, 18965, 19140, 45430, 47005, 75100 et celui dénommé Lactoflavine ; les colorants oranges identifiés par les numéros de Color Index n° 14270, 15510, 15980, 15985, 16230, 20170, 40215 ; les colorants rouges identifiés par les numéros de Color Index n° 14700, 14720, 14815, 15620, 16035, 16185, 16255, 16290, 17200, 18050, 18130, 18736, 24790, 27290, 45100, 45220, 45380, 45405, 45410, 45425, 45430, 75470 et leurs mélanges.

8. Procédé selon la revendication 5, 6 ou 7, **caractérisé en ce que** le colorant liposoluble est choisi parmi le colorant brun identifié sous le numéro Color Index n° 12010 ; les colorants jaunes identifiés respectivement par les numéros de Color Index n° 12700, 21230, 47000, 75125, 75135 ; les colorants oranges identifiés sous les numéros de Color Index n° 11920, 40800, 40820, 40825, 40850, 45396, 75120, 75130 et la capasanthine et le colorant rouge identifié par le numéro 12150 et leurs mélanges.

9. Procédé selon l'une quelconque des revendications précédentes, **caractérisé en ce que** lesdits agents de coloration sont traités en surface.

10. Procédé selon l'une quelconque des revendications précédentes, **caractérisé en ce que** la nacre est choisie parmi les nacres de couleur or, les nacres bronzes, les nacres oranges, les nacres de teinte brune, les nacres à reflet cuivre, les nacres à reflet rouge, les nacres à reflet jaune, les nacres de teinte rouge à reflet or, les nacres roses, les nacres noires à reflet or, les nacres bleues, les nacres orangées, rosées, vert doré et leurs mélanges.

11. Procédé selon l'une quelconque des revendications précédentes, **caractérisé en ce que** la composition comprend en outre, à titre de particules réfléchissantes, au moins un agent de coloration goniochromatique.

12. Procédé selon l'une quelconque des revendications précédentes, **caractérisé en ce que** ladite composition comprend au moins une phase aqueuse.

13. Procédé selon l'une quelconque des revendications précédentes, **caractérisé en ce que** ladite composition comprend au moins une phase grasse.

14. Procédé selon la revendication 13, **caractérisé en ce que** ladite composition est anhydre.

15. Procédé selon la revendication 13 ou 14, **caractérisé en ce que** ladite phase grasse contient au moins un corps gras liquide à température ambiante et à pression atmosphérique et/ou au moins un corps gras solide à température ambiante et à pression atmosphérique.

16. Procédé selon la revendication 15, **caractérisé en ce que** ledit corps gras liquide à température ambiante et à pression atmosphérique comprend au moins une huile volatile ou non volatile ou un de leurs mélanges.

17. Procédé selon la revendication 15 ou 16, **caractérisé en ce que** ledit corps gras solide à température ambiante et pression atmosphérique est choisi parmi les cires, les corps gras pâteux, les gommes et leurs mélanges.

18. Procédé selon l'une quelconque des revendications précédentes, **caractérisé en ce que** ladite composition comprend en outre au moins une charge complémentaire.

19. Procédé selon l'une quelconque des revendications précédentes, **caractérisé en ce que** ladite composition comprend en outre au moins un polymère filmogène.

20. Procédé selon l'une quelconque des revendications précédentes, **caractérisé en ce que** ladite composition se présente sous une forme fluide de type liquide, pâte, émulsion directe ou inverse, ou gel.

21. Procédé selon l'une quelconque des revendications 1 à 19, **caractérisé en ce que** ladite composition se présente sous une forme solide notamment compacte, pulvérulente ou coulée ou sous forme de stick.

22. Procédé selon l'une quelconque des revendications précédentes, **caractérisé en ce que** ladite composition se présente sous la forme d'un fond de teint à appliquer sur le visage ou le cou, d'un produit anti-cernes, d'un correcteur de teint, d'une crème teintée ou d'une base de maquillage pour le visage ou d'une composition de maquillage pour le corps.

23. Procédé de maquillage d'une peau dont la clarté moyenne L* mesurée sur le front, les pommettes et le menton dans l'espace colorimétrique CIE 1976, est inférieure à 60, **caractérisé en ce qu'**il comprend l'application sur ladite peau d'au moins un produit de type fond de teint, comprenant au moins une première et une seconde compositions, chacune dans un contenant, la première composition comprenant, dans un premier milieu physiologiquement acceptable, au moins un agent de coloration possédant une réfléctance significative d'une coloration jaune ou orangée dans l'intervalle 550 à 675 nm et la seconde composition comprenant, dans un second milieu physiologiquement acceptable, au moins une nacre à titre de particules réfléchissantes.

24. Procédé selon la revendication 23, **caractérisé en ce que** l'agent de coloration et les particules réfléchissantes sont tels que définis en revendications 1 à 11.

25. Procédé selon la revendication 23 ou 24, **caractérisé en ce qu'**il comprend l'application d'une première couche de l'une des deux compositions dudit produit dite composition de base puis, l'application sur au moins une partie de ladite première couche, d'une deuxième couche de l'autre composition dudit produit dite composition de surface.

## Claims

1. Process for making up skin whose mean lightness L*, measured on the forehead, the cheekbones and the chin, in the CIE 1976 colorimetric space, is less than 60, **characterized in that** it comprises the application to the said skin of at least one composition of foundation type combining, in a physiologically acceptable medium, at least one colouring agent having significant reflectance of a yellow or orange coloration in the range 550 to 675 nm, and at least one nacre as reflective particles, this combination being capable of giving the said composition a hue angle h ranging from 40° to 70° and especially from 50° to 70°, and a saturation C* ranging from 20 to 50.

2. Process according to Claim 1, **characterized in that** the said composition comprises from 0.5% to 30%, especially from 2% to 20% and in particular from 5% to 18% by weight of colouring agent(s) relative to its total weight.

3. Process according to either of the preceding claims, **characterized in that** the said composition comprises from 0.5% to 60% by weight, especially from 1% to 30% by weight, in particular from 2% to 20% and more particularly from 3% to 10% by weight of reflective particles, relative to its total weight.

4. Process according to any one of the preceding claims, **characterized in that** the reflective particles have a yellow, pink, red, bronze, orange, brown, gold and/or coppery colour or tint.

5. Process according to any one of the preceding claims, **characterized in that** the said colouring agent is chosen from mineral or organic pigments, colouring polymers, water-soluble or liposoluble dyes, organic lakes and metallic powders, and mixtures thereof.

6. Process according to Claim 5, **characterized in that** the mineral pigments are chosen from yellow, red and brown metal oxides and especially iron oxides.

7. Process according to Claim 5 or 6, **characterized in that** the water-soluble dye is chosen from the brown dye identified by the name "caramel" according to the Colour Index; the yellow dyes identified by the Colour Index numbers 10316, 13015, 18690, 18820, 18965, 19140, 45430, 47005, 75100 and that known as Lactoflavin; the orange dyes identified by the Colour Index numbers 14270, 15510, 15980, 15985, 16230, 20170, 40215; the red dyes identified by the Colour Index numbers 14700, 14720, 14815, 15620, 16035, 16185, 16255, 16290, 17200, 18050, 18130, 18736, 24790, 27290, 45100, 45220, 45380, 45405, 45410, 45425, 45430, 75470, and mixtures thereof.

8. Process according to Claim 5, 6 or 7, **characterized in that** the liposoluble dye is chosen from the brown dye identified by the Colour Index number 12010; the yellow dyes identified, respectively, by the Colour Index numbers 12700, 21230, 47000, 75125, 75135; the orange dyes identified by the Colour Index numbers 11920, 40800, 40820, 40825, 40850, 45396, 75120, 75130 and capsanthin and the red dye identified by number 12150, and mixtures thereof.

9. Process according to any one of the preceding claims, **characterized in that** the said colouring agents are surface-treated.

10. Process according to any one of the preceding claims, **characterized in that** the nacre is chosen from nacres of golden colour, bronze nacres, orange nacres, nacres with a brown tint, nacres with a coppery tint, nacres with a red tint, nacres with a yellow tint, nacres of red hue with a golden tint, pink nacres, black nacres with a golden tint, blue nacres and, orange, pink and golden-green nacres, and mixtures thereof.

11. Process according to any one of the preceding claims, **characterized in that** the composition also comprises, as reflective particles, at least one goniochromatic colouring agent.

12. Process according to any one of the preceding claims, **characterized in that** the said composition comprises at least one aqueous phase.

13. Process according to any one of the preceding claims, **characterized in that** the said composition comprises at least one fatty phase.

14. Process according to Claim 13, **characterized in that** the said composition is anhydrous.

15. Process according to Claim 13 or 14, **characterized in that** the said fatty phase contains at least one fatty substance that is liquid at room temperature and at atmospheric pressure and/or at least one fatty substance that is solid at room temperature and at atmospheric pressure.

16. Process according to Claim 15, **characterized in that** the said fatty substance that is liquid at room temperature and at atmospheric pressure comprises at least one volatile or non-volatile oil or a mixture thereof.

17. Process according to Claim 15 or 16, **characterized in that** the said fatty substance that is solid at room temperature and atmospheric pressure is chosen from waxes, pasty fatty substances and gums, and mixtures thereof.

18. Process according to any one of the preceding claims, **characterized in that** the said composition also comprises at least one additional filler.

19. Process according to any one of the preceding claims, **characterized in that** the said composition also comprises at least one film-forming polymer.

20. Process according to any one of the preceding claims, **characterized in that** the said composition is in a fluid form of liquid, paste, direct or inverse emulsion or gel type.

21. Process according to any one of Claims 1 to 19, **characterized in that** the said composition is in a solid and especially compact, pulverulent or cast form or in the form of a stick.

22. Process according to any one of the preceding claims, **characterized in that** the said composition is in the form of a foundation to be applied to the face or the neck, a concealer product, a complexion corrector, a tinted cream or makeup base for the face or a makeup composition for the body.

23. Process for making up skin whose mean lightness L*, measured on the forehead, the cheekbones and the chin, in the CIE 1976 colorimetric space, is less than 60, **characterized in that** it comprises the application to the said skin of at least one product of foundation type, comprising at least one first and one second composition, each in a container, the first composition comprising, in a first physiologically acceptable medium, at least one colouring agent having significant reflectance of a yellow or orange coloration in the range 550 to 675 nm, and the second composition comprising, in a second physiologically acceptable medium, at least one nacre as reflective particles.

24. Process according to Claim 23, **characterized in that** the colouring agent and the reflective particles are as defined in Claims 1 to 11.

25. Process according to Claim 23 or 24, **characterized in that** it comprises the application of a first coat of one of the two compositions of the said product, referred to as the base composition, followed by the application, onto at least part of the said first coat, of a second coat of the other composition of the said product, referred to as the surface composition.

## Patentansprüche

1. Verfahren zum Schminken einer Haut, deren auf der Stirn, den Backenknochen und dem Kinn gemessene mittlere Helligkeit L* im kolorimetrischen Raum CIE 1976 weniger als 60 beträgt, **dadurch gekennzeichnet, dass** es das Auftragen mindestens einer Zusammensetzung vom Typ Teintgrundierung auf diese Haut umfasst, die in einem physiologisch annehmbaren Medium mindestens ein Färbemittel mit einem signifikanten Reflexionsvermögen von einer gelben oder orangefarbenen Farbe im Intervall 550 bis 675 nm und mindestens ein Perlmutt als reflektierende Teilchen kombiniert, wobei diese Kombination in der Lage ist, dieser Zusammensetzung einen Bunttonwinkel h von 40° bis 70°, insbesondere von 50° bis 70°, und eine Sättigung C* von 20 bis 50 zu verleihen.

2. Verfahren nach Anspruch 1, **dadurch gekennzeichnet, dass** die Zusammensetzung 0,5 bis 30 Gew.-%, insbesondere 2 bis 20 Gew.-% und im Besonderen 5 bis 18 Gew.-% Färbemittel bezüglich ihres Gesamtgewichts umfasst.

3. Verfahren nach einem der vorhergehenden Ansprüche, **dadurch gekennzeichnet, dass** die Zusammensetzung 0,5 bis 60 Gew.-%, insbesondere 1 bis 30 Gew.-%, im Besonderen 2 bis 20 Gew.-% reflektierende Teilchen und ganz besonders 3 bis 10 Gew.-% bezüglich ihres Gesamtgewichts umfasst.

4. Verfahren nach einem der vorhergehenden Ansprüche, **dadurch gekennzeichnet, dass** die reflektierenden Teilchen eine gelben, rosafarbenen, roten, bronzefarbenen, orangefarbenen, braunen, goldenen und/oder kupfrigen Farbton oder Glanz besitzen.

5. Verfahren nach einem der vorhergehenden Ansprüche, **dadurch gekennzeichnet, dass** das Färbemittel aus den mineralischen oder organischen Pigmenten, färbenden Polymeren, den wasserlöslichen oder fettlöslichen Farbstoffen, den organischen Lacken, den Metallpulvern und ihren Mischungen ausgewählt ist.

6. Verfahren nach Anspruch 5, **dadurch gekennzeichnet, dass** die mineralischen Pigmente aus den gelben, roten, braunen Metalloxiden und insbesondere den Eisenoxiden ausgewählt sind.

7. Verfahren nach Anspruch 5 oder 6, **dadurch gekennzeichnet, dass** der wasserlösliche Farbstoff ausgewählt ist aus dem braunen Farbstoff, der nach dem Color Index mit der Bezeichnung "Karamell" identifiziert ist; den gelben Farbstoffen, die durch die Color-Index-Nummern 10316, 13015, 18690, 18820, 18965, 19140, 45430, 47005, 75100 identifiziert sind, und demjenigen, der Lactoflavin genannt wird; den orangefarbenen Farbstoffen, die durch die Color-Index-Nummern 14270, 15510, 15980, 15985, 16230, 20170, 40215 identifiziert sind; den roten Farbstoffen, die durch die Color-Index-Nummern 14700, 14720, 14815, 15620, 16035, 16185, 16255, 16290, 17200, 18050, 18130, 18736, 24790, 27290, 45100, 45220, 45380, 45405, 45410, 45425, 45430,75470 identifiziert sind, und ihren Mischungen.

8. Verfahren nach Anspruch 5, 6 oder 7, **dadurch gekennzeichnet, dass** der fettlösliche Farbstoff ausgewählt ist aus dem braunen Farbstoff, der durch die Color-Index-Nummer 12010 identifiziert ist; den gelben Farbstoffen, die durch die Color-Index-Nummern 12700, 21230, 47000, 75125, 75135 identifiziert sind; den orangefarbenen Farbstoffen, die durch die Color-Index-Nummern 11920, 40800, 40820, 40825, 40850, 45396, 75120, 75130 identifiziert sind, und Capasanthin und dem roten Farbstoff, der durch die Nummer 12150 identifiziert ist, und ihren Mischungen.

9. Verfahren nach einem der vorhergehenden Ansprüche, **dadurch gekennzeichnet, dass** die Färbemittel oberflächenbehandelt sind.

10. Verfahren nach einem der vorhergehenden Ansprüche, **dadurch gekennzeichnet, dass** das Perlmutt ausgewählt ist aus den Perlmuttarten von goldener Farbe, den bronzefarbenen Perlmuttarten, den orangefarbenen Perlmuttarten, den Perlmuttarten mit braunem Farbton, den Perlmuttarten mit Kupferglanz, den Perlmuttarten mit rotem Glanz, den Perlmuttarten mit gelbem Glanz, den Perlmuttarten mit rotem Farbton mit Goldglanz, den rosafarbenen Perlmuttarten, den schwarzen Perlmuttarten mit Goldglanz, den blauen Perlmuttarten, den orangefarbenen, rosafarbenen, goldgrünen Perlmuttarten und ihren Mischungen.

11. Verfahren nach einem der vorhergehenden Ansprüche, **dadurch gekennzeichnet, dass** die Zusammensetzung außerdem als reflektierende Teilchen mindestens ein goniochromatisches Färbemittel umfasst.

12. Verfahren nach einem der vorhergehenden Ansprüche, **dadurch gekennzeichnet, dass** die Zusammensetzung mindestens eine wässrige Phase umfasst.

13. Verfahren nach einem der vorhergehenden Ansprüche, **dadurch gekennzeichnet, dass** diese Zusammensetzung mindestens eine Fettphase umfasst.

14. Verfahren nach Anspruch 13, **dadurch gekennzeichnet, dass** diese Zusammensetzung wasserfrei ist.

15. Verfahren nach Anspruch 13 oder 14, **dadurch gekennzeichnet, dass** die Fettphase mindestens ein bei Umgebungstemperatur und bei atmosphärischem Druck flüssiges Fett und/oder mindestens ein bei Umgebungstemperatur und bei atmosphärischem Druck festes Fett enthält.

16. Verfahren nach Anspruch 15, **dadurch gekennzeichnet, dass** das bei Umgebungstemperatur und bei atmosphärischem Druck flüssige Fett mindestens ein flüchtiges oder nicht flüchtiges Öl oder eine ihrer Mischungen umfasst.

17. Verfahren nach Anspruch 15 oder 16, **dadurch gekennzeichnet, dass** das bei Umgebungstemperatur und bei atmosphärischem Druck feste Fett aus den Wachsen, den breiförmigen Fetten, den Gummen und ihren Mischungen ausgewählt ist.

18. Verfahren nach einem der vorhergehenden Ansprüche, **dadurch gekennzeichnet, dass** diese Zusammensetzung außerdem mindestens eine ergänzende Charge umfasst.

19. Verfahren nach einem der vorhergehenden Ansprüche, **dadurch gekennzeichnet, dass** die Zusammensetzung mindestens ein filmbildendes Polymer umfasst.

20. Verfahren nach einem der vorhergehenden Ansprüche, **dadurch gekennzeichnet, das** die Zusammensetzung in einer fließfähigen Form vom Typ Flüssigkeit, Brei, direkte oder inverse Emulsion oder Gel vorliegt.

21. Verfahren nach einem der Ansprüche 1 bis 19, **dadurch gekennzeichnet, dass** die Zusammensetzung in einer festen Form, insbesondere einer kompakten, pulverförmigen oder gegossenen Form oder in Form eines Sticks vorliegt.

22. Verfahren nach einem der vorhergehenden Ansprüche, **dadurch gekennzeichnet, dass** die Zusammensetzung in der Form einer auf das Gesicht oder den Hals aufzutragenden Teintgrundierung, eines Produkts gegen Ringe, einer Teintkorrektur, einer getönten Creme oder einer Schminkgrundlage für das Gesicht oder einer Schminkzusammensetzung für den Körper vorliegt.

23. Verfahren zum Schminken einer Haut deren auf der Stirn, den Backenknochen und dem Kinn gemessene mittlere Helligkeit L* im kolorimetrischen Farbraum CIE 1976 weniger als 60 beträgt, **dadurch gekennzeichnet, dass** es das Auftragen mindestens eines Produkts vom Typ Teintgrundierung auf die Haut umfasst, das mindestens eine erste und eine zweite Zusammensetzung jeweils in einem Behältnis umfasst, wobei die erste Zusammensetzung in einem ersten physiologisch annehmbaren Medium mindestens ein Färbemittel mit einem signifikanten Reflexionsvermögen von einer gelben oder orangefarbenen Farbe im Intervall von 550 bis 675 nm umfasst und die zweite Zusammensetzung in einem zweiten physiologisch annehmbaren Medium mindestens ein Perlmutt als reflektierende Teilchen umfasst.

24. Verfahren nach Anspruch 23, **dadurch gekennzeichnet, dass** das Färbemittel und die reflektierenden Teilchen so sind, wie sie in den Ansprüchen 1 bis 11 definiert sind.

25. Verfahren nach Anspruch 23 oder 24, **dadurch gekennzeichnet, dass** es das Auftragen einer ersten Schicht einer der beiden Zusammensetzungen dieses Produkts, Basiszusammensetzung genannt, und dann das Auftragen einer zweiten Schicht der anderen Zusammensetzung dieses Produkts, Oberflächenzusammensetzung genannt, auf mindestens einen Teil der ersten Schicht umfasst.
